# EUROPEAN PATENT APPLICATION

(11) **EP 2 856 946 A1**
(43) Date of publication of application: **08.04.2015**
(21) Application number: 13187614.6
(22) Date of filing: 07.10.2013
(51) Int. Cl.: A61B 17/00, A61B 17/12

(54) **A medical implant for occluding an opening in a body and a method of producing such a medical implant**

(71) Applicant: Occlutech Holding AG, 8201 Schaffhausen (CH)
(72) Inventor: Nielsen, Stevan, 72108 Rottenburg Am Neckar (DE)
(74) Representative: KIPA AB

(57) **Abstract**

The disclosure relates to a medical implant (1) for occluding an opening in a body and a method of producing such a medical implant. Disclosed is an improved occluder, which provides improved occlusion, improved sealing and improved endothelialization. A method is disclosed comprising braiding, knitting or weaving together strands to form a body mesh of strands forming a plurality of adjacent cells delimited by the strands. The method further comprises applying a first coating to said strands, and applying a second coating to at least part of an external surface of said medical implant, whereby the ratio between the amount of the first and second coating on said external surface is different from said ratio on an internal surface of the implant.

## Description

### Field of the invention

This disclosure pertains in general to the field of medical implants. More particularly, the disclosure relates to occluding devices or occluders. In particular the disclosure relates to applying of a coating to a surface of a medical implant or to cover an outside of a medical implant with a coating or covering for improving the sealing of a defect, such as a defect in the heart.

### Background of the invention

An occluder is a medical product or implant used for occluding, i.e. closing, defects e.g. in the human heart. Defects may occur in various regions of the heart and have different forms. Defects in the septum of the atrium are common. The occluders can be inserted using minimally invasive cardiac catheter techniques, more precisely by means of a transvenous, catheter-interventional access. One type of occluders is made of Nitinol threads, e.g. having a double disc shape with an intermediate tubular section, having a smaller diameter than the discs, between the discs. They are inserted in openings that are to be closed, one disc on each side of the hole, which is to be closed and with the intermediate tubular section in the center of the hole, the discs being larger than the hole. There are two examples of such devices. The first, made by Occlutech^{®} having one fixation point at the end of the device and the second, made by AGA medical^{®} having two fixation points, one at each end of the device. In these devices, the Nitinol threads are joined in the centre of one or both of the discs.

These occluders normally comprise some kind of mesh and may be provided with a polyester fabric inside the mesh for limiting the blood flow through the occluder. An example of such a prior art polyester fabric is given in the published US application US2009/0082803 A1.

Furthermore, WO2004/082532 Al discloses a membrane attached outside an occluder.

However, an occluder having an internal fabric or a membrane attached outside may be difficult and time-consuming to produce. Moreover, the use of artificial fibrous material in an occluder may lead to blood clotting or thrombosis. If the clotting is too severe and the clot breaks free, the clot may travel to other parts of the body. Such travelling clots are known as embolus. Emboli are carried by the circulation and capable of clogging arterial capillary beds at a site different from its point of origin, i.e. they can create an unwanted arterial occlusion or vascular occlusion. When vascular occlusion occurs in a major vein, it can cause deep vein thrombosis. Deep vein thrombosis commonly affects the leg veins, e.g. the femoral vein or the popliteal vein, or the veins in the pelvis. The most serious complication of a deep vein thrombosis is that the clot could dislodge and travel to the lungs, which is called a pulmonary embolism. There, in the lungs, the clots can block the main artery of the lungs. Previous methods for providing a coating on the occluder may also need to be improved to achieve improved properties of the occluder, which improvement include to optimize the coating to achive the desired function of the occluder and to provide for the ability to optimize to different applications and procedures. Furthermore, previous methods for providing blood flow limiting means on the outside of the occluder may also need to be improved to achieve improved occluding ability of the occluder. Moreover, previous methods providing blood flow limiting means on the outside of the occluder are complex and time consuming.

Thus, it would be advantageous to provide an improved method of manufacturing an occluder with blood flow limiting means for providing the above mentioned improvements.

### Summary of the invention

Accordingly, embodiments of the present disclosure preferably seek to mitigate, alleviate or eliminate one or more deficiencies, disadvantages or issues in the art, such as the above-identified, singly or in any combination by providing a medical implant for occluding an opening in a body and a method of producing such a medical implant, according to the appended patent claims.

According to aspects of the disclosure, a medical implant and a method of producing such a medical implant are disclosed, wherein the medical implant is covered with a coating for sealing of a defect, such as a defect in the heart.

According to a first aspect of the disclosure, a method of producing a medical implant for occluding an opening in a body is provided. The method comprises braiding, knitting or weaving together strands to form a body mesh of strands forming a plurality of adjacent cells delimited by the strands. The method further comprises applying a first coating to said strands, and applying a second coating to at least part of an external surface of said medical implant, whereby the ratio between the amount of the first and second coating on said external surface is different from said ratio on an internal surface of the implant.

According to a second aspect of the disclosure, a medical implant, such as an atrial septal defect (ASD) occluder, a Patent foramen ovale (PFO) occluder, a paravalvular leakage (PLD) occluder, a PDA occluder, an LAA occluder, a ventricular septal defect (VSD) occluder, or a transapical occluder, for occluding an opening in a body, wherein said medical implant comprises a body mesh of strands, said strands being braided, knitted or woven together to form the mesh, said body mesh forming a plurality of adjacent cells delimited by said strands, said body mesh having an external surface, and a first coating on said strands, and a second coating covering at least part of said external surface for at least partly restricting a fluid flow through a structural tissue defect, such as a defect in the heart, whereby the ratio between the amount of the first and second coating on said external surface is different from said ratio on an internal surface of the implant.

Further embodiments of the disclosure are defined in the dependent claims, wherein features for the second and any other aspects of the disclosure are as for the first aspect mutatis mutandis.

The use of a coating outside an external surface of a medical implant provides for a lower friction of the medical implant in e.g. a catheter.

Some embodiments of the disclosure provide for improved properties of the occluder, which improvement include to optimize the coating to achive a desired function of the occluder and to provide for the ability to coat the occluder with a larger variety of different coatings for optimization to different applications and procedures.

Some embodiments of the disclosure provide for improving the method by which blood flow limiting means are provided on the outside of the occluder for achieving improved occluding ability of the occluder.

Some embodiments of the disclosure provide for manufacturing an occluder with blood flow limiting means on the outside of the occluder in a more simple and efficient manner.

Some embodiments of the disclosure also provide for an improved occlusion.

Some embodiments of the disclosure also provide for improved sealing of a defect, such as a heart defect.

Some embodiments of the disclosure also provide for an improved endothelialization.

Some embodiments of the disclosure also provide for slowing down the blood flow through the defect.

Some embodiments of the disclosure also provide for an advantageous and/or easier delivery of the medical implant, since the use of a coating outside an external surface of a medical implant may make the medical implant glide or slide easier through a delivery catheter.

Some embodiments of the disclosure also provide for enabling an initial controllable fluid retention.

Some embodiments of the disclosure also provide for that an inflow of blood to different areas of a medical implant is controlled or controllable.

Some embodiments of the disclosure also provide for that the flow is efficiently restricted by covering at least substantially the full diameter of both ends of the medical implant.

Some embodiments of the disclosure also provide for that integration of the medical implant with surrounding blood is enhanced.

Some embodiments of the disclosure also provide for that the coating or covering is free of tension, so that pre-mature fatigue thereof can be avoided and thus a reliable ingrowth is allowed for.

Some embodiments of the disclosure also contribute to facilitation of expansion into an expanded shape, since the coating elastically contributes to expansion into the expanded shape, i.e. by making the coating elastic and by applying the coating to the medical implant, while the medical implant is in its expanded shape, the coating on the external surface of the medical implant is prone to contribute to force the medical implant into its expanded shape.

Some embodiments of the disclosure also provide for facilitation of the delivery of the medical implant through a catheter, since the coating is prone to contribute to force the medical implant into its contracted shape if the coating is applied to the medical implant while the medical implant is in its contracted shape.

Some embodiments of the disclosure also provide for that the occlusion is not abrupt upon implantation.

Some embodiments of the disclosure also provide for that a certain blood flow may still occur after implantation and gradually decline upon blood coagulation and/or endothelialization of the implanted medical implant.

Some embodiments of the disclosure also provide for that friction of the medical device is lowered, e.g. during delivery through a catheter.

Some embodiments of the disclosure also provide for that cellular biocompatibility is maximized.

Some embodiments of the disclosure also provide for a less time consuming manufacturing of a medical implant.

Some embodiments of the disclosure also provide for a very flexible medical implant.

Some embodiments of the disclosure also provide for a medical implant with a particularly large expansion/contraction ratio.

Some embodiments of the disclosure provides for easier and cheaper manufacturing.

Some embodiments of the disclosure provides for that the inflow to different areas of the medical implant can be controlled, not just stopped.

Some embodiments of the disclosure provide for that no damage is done to body tissue by elements used for securing the position of the medical implant, since no barbs or hooks are used for this purpose.

Some embodiments of the disclosure provide for that pericardial effusion is avoided.

It should be emphasized that the term "comprises/comprising" when used in this specification is taken to specify the presence of stated features, integers, steps or components but does not preclude the presence or addition of one or more other features, integers, steps, components or groups thereof.

### Brief description of the drawings

These and other aspects, features and advantages of which embodiments of the disclosure are capable of will be apparent and elucidated from the following description of embodiments of the present disclosure, reference being made to the accompanying drawings, in which;
Fig. 1 is a lateral view of a medical implant provided with coating by a method according to an embodiment of the invention;
Fig. 2a is a magnified view of section (A) in Fig. 1;
Figs. 2b-c illustrates the part of the implant in Fig. 2a having been provided with coating according to an embodiment of the invention;
Figs. 3a-c illustrates a part of the implant having been provided with coating according to another embodiment of the invention;
Figs. 4a-b illustrates a part of the implant having been provided with coating according to another embodiment of the invention as seen from the interior of the implant;
Figs. 5a-b illustrates a part of the implant having been provided with coating according to another embodiment of the invention as seen from the exterior of the implant;
Fig. 6 illustrates a method according to embodiments of the invention;
Figs. 7a-d is a top view of a medical implant having a coating with different perforations according to embodiments of the invention;
Fig. 8 is a top view of a medical implant with a coating according to an embodiment of the invention;
Fig. 9 is a lateral view of a medical implant, which has been provided with a coating at both ends;
Fig. 10 is a top view of a medical implant with a coating according to an embodiment of the invention;
Fig. 11 is a lateral view of a medical implant being coated by dipping;
Fig. 12 is a lateral view of a medical implant being coated by spraying;
Fig. 13 is a schematic sketch of a medical implant being coated by a process, such as electro-spinning or Nano-spinning;
Fig. 14 is a further view of an implant according to an embodiment of the invention, being covered with a coating according to a method of the invention.

### Description of embodiments

Specific embodiments of the disclosure now will be described with reference to the accompanying drawings. This disclosure may, however, be embodied in many different forms and should not be construed as limited to the embodiments set forth herein; rather, these embodiments are provided so that this disclosure will be thorough and complete, and will fully convey the scope of the disclosure to those skilled in the art. The terminology used in the detailed description of the embodiments illustrated in the accompanying drawings is not intended to be limiting of the disclosure. In the drawings, like numbers refer to like elements.

The following description focuses on an embodiment of the present disclosure applicable to occluders such as an atrial septal defect (ASD) occluder, a Patent foramen ovale (PFO) occluder, a paravalvular leakage (PLD) occluder, a PDA occluder, an LAA occluder, a ventricular septal defect (VSD) occluder, or a transapical occlude. However, it will be appreciated that the disclosure is not limited to this application but may be applied to many other medical implants including for example Filters, Stents, Vascular Occluders, Products for treatment of aneurysm, Plugs and Occlusion systems for other applications.

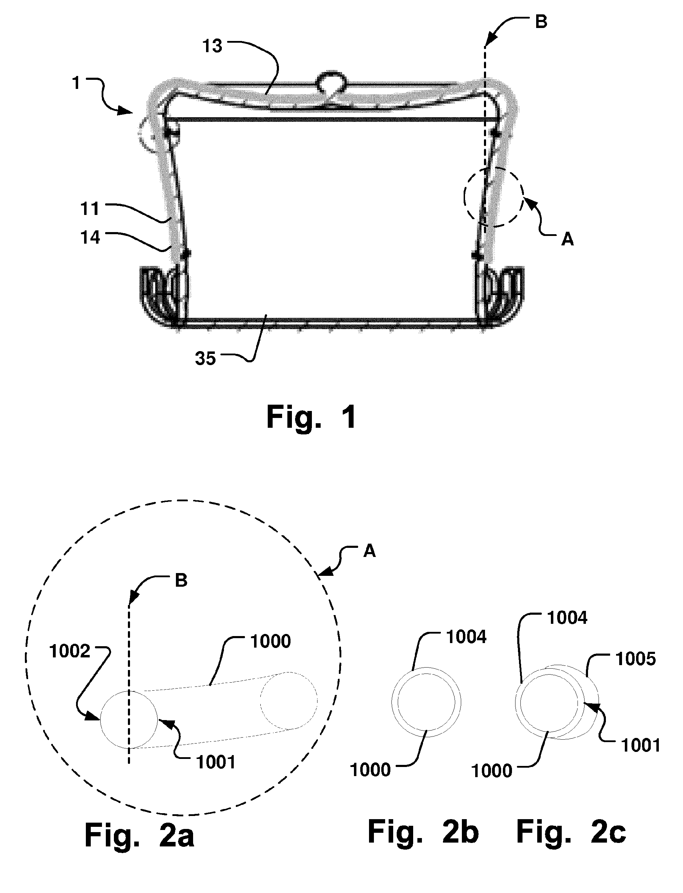

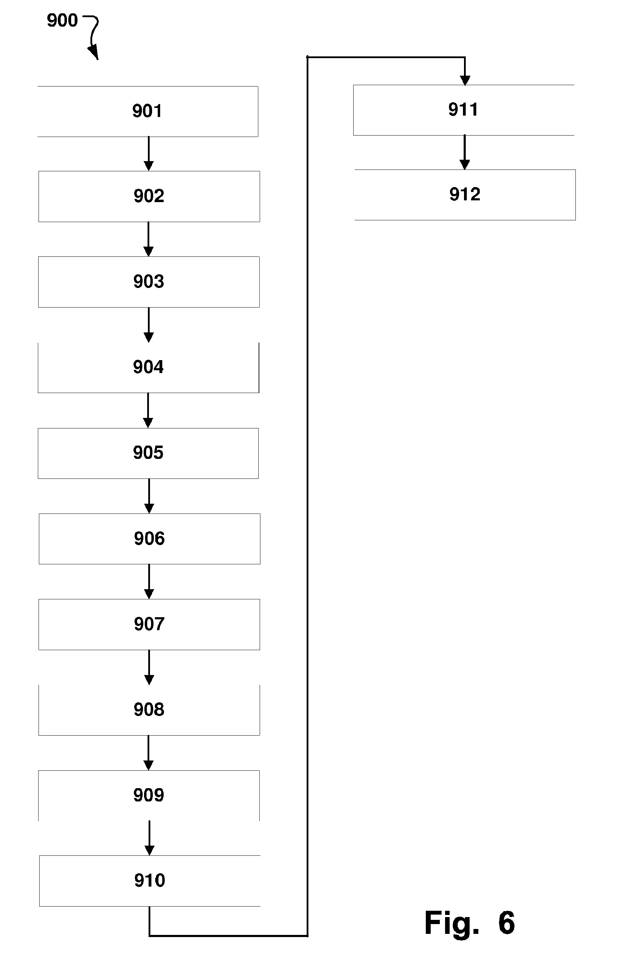

Fig. 1 shows a medical implant 1 that is provided with a coating 11. The coating 11 may comprise a first 1004 and/or a second coating 1005 as described below. A method 900 of producing a medical implant 1 for occluding an opening in a body according to embodiments of the invention is illustrated in Figs. 2a-c, 3a-c, and 6. The method 900 comprises braiding, knitting or weaving 901 together strands 1000 to form a body mesh of strands and forming a plurality of adjacent cells delimited by said strands, and applying 902 a first coating 1004 to said strands, and applying 904 a second coating 1005 to at least part of an external surface 1001 of said medical implant 1, whereby the ratio between the amount of the first and second coating on said external surface 1001 is different from said ratio on an internal surface 1002 of the implant 1. Hence it is provided for a medical implant where the interior and exterior surfaces of the implant, which may be defined by the surface of the threads 1000 facing the interior or exterior of the implant respectively, have different properties since it the method 900 provides for selectiveness in the amount of coated material, and choice of material, on the interior 1002 and exterior 1001 surfaces respectively. The selective coating allows for example that the material of the first coating 1004 can be optimized for biocompatibility, and the second coating 1005 for providing the occluding ability of the implant 1, by reducing the area defined by the cells between the strands. By having different material compositions, such as a varying ratios of the first 1004 and second coatings 1005 on the interior and exterior surfaces different functionalization of the surfaces is possible, e.g. having a biocompatible interior surface 1002 combined with drug delivery or occluding properties of the exterior surface 1001. The ratio can be zero on each of the surfaces, thus the interior surface may only be provided with the first coating 1004 and the exterior surface may only be provided the second coating 1005. The ratio may be in the range of 0 to 1, for example, the exterior surface can have a ratio of 1, by which equal amounts of the first and second coating are provided on the exterior surface, or any other ratio, while the interior surface has a different ratio such as zero. Hence by providing for the ability to have such varying ratios the total amount of material can be reduced in addition to allowing for optimization and differentiation in surface functionality while providing for the discussed selectivity. Providing for selected functionality while at the same time reducing the amount of coating material allows for e.g. fewer complications due to interference of the coating material or undesired influence of the coating material on structural or functional properties on the implant 1 can be minimized or avoided. Interaction between the first and second coating may provide for a desired synergy and functionality of the implant to improve e.g. occluding ability, structural integrity, e.g. extension of the implant's lifetime, and biocompatibility.

Fig. 2a is a magnified cut-out view of section A as seen in Fig. 1, illustrating a strand 1000 of the implant 1. The interior 1002 and exterior surfaces 1001 of the implant 1 are illustrated by the dividing line B in the Figs. 1 and 2a, where the latter figure illustrates how each strand 1000 will have an interior 1002 and exterior surface 1001 facing the interior and exterior of the implant 1 respectively. The plurality of strands 1000 forming the implant 1 will hence define the interior 1002 and exterior surface 1001 of the implant 1. Fig. 2b illustrates first coating 1004 applied to the strand 1000, and Fig. 2c illustrates the second coating 1005 applied to the external surface 1001.

As mentioned above the second coating 1005 may be applied to the external surface 1001 only, whereby the aforementioned ratio is zero on the internal surface 1002. Such selectivity may be advantageous when the second coating is primarily provided for interaction with the surrounding tissue of the implanted device 1, hence keeping the amount of material on the interior of the implant at a minimum for optimizing biocompatibility and avoiding interference of the coating material with the anatomy or blood flow, while allowing for e.g. increasing the occluding ability via the second coating.

The second coating 1005 may be applied subsequent of applying the first coating 1004. While the second coating may be optimized for a desired property for interaction with the surrounding anatomy, the first coating may be provided as an optimized interface between the second coating 1005 and each of the strands 1000. A larger variety of coatings can be applied to the external surface 1001, which may be difficult to fixate to the strands 1000, since the first coating 1004 functions as an intermediate interface between the second coating and the strands 1000, such as an adhesion layer. Thus it is sufficient for the second coating to be compatible with the first coating, and not necessarily with the material of the strands 1000. The first coating may be a non-fibrous film or layer 1003 of polymer molecules providing for subsequent adhesion to the second coating 1005.

The second coating may be applied subsequent of drying 903 of the first coating. This may improve the interaction between the first and second coating, such as improving adhesion there between. The first coating may comprise a polymer. Thus drying the first coating may leave a residual non-fibrous layer 1003 of polymer molecules on the implant for subsequent adhesion to the second coating 1005. Thus the first coating may provide a binding force to the second coating such that the second coating adheres to the strands.

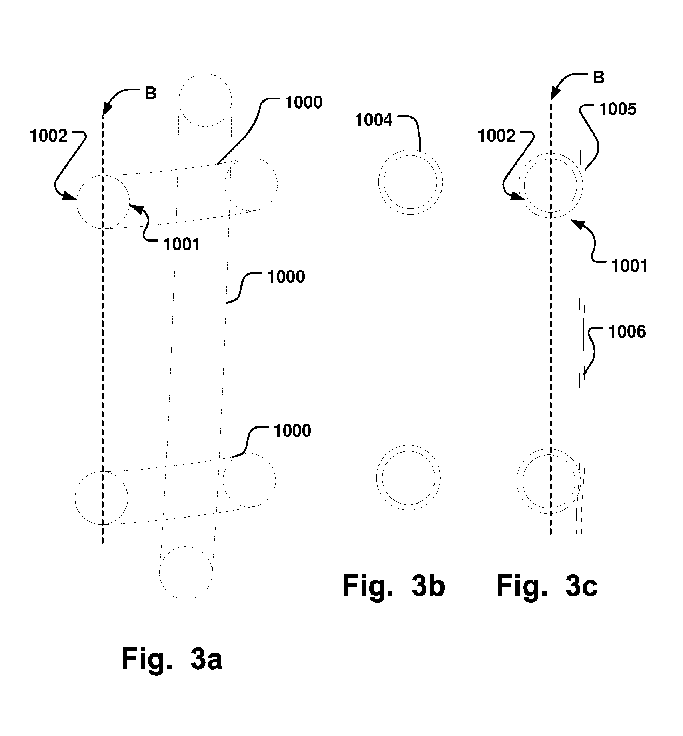

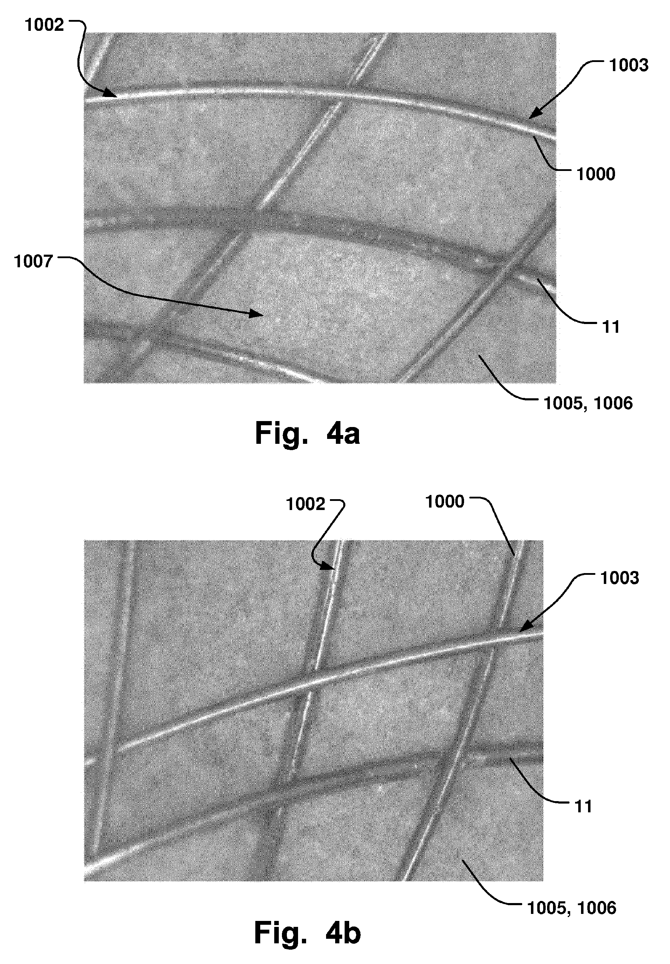

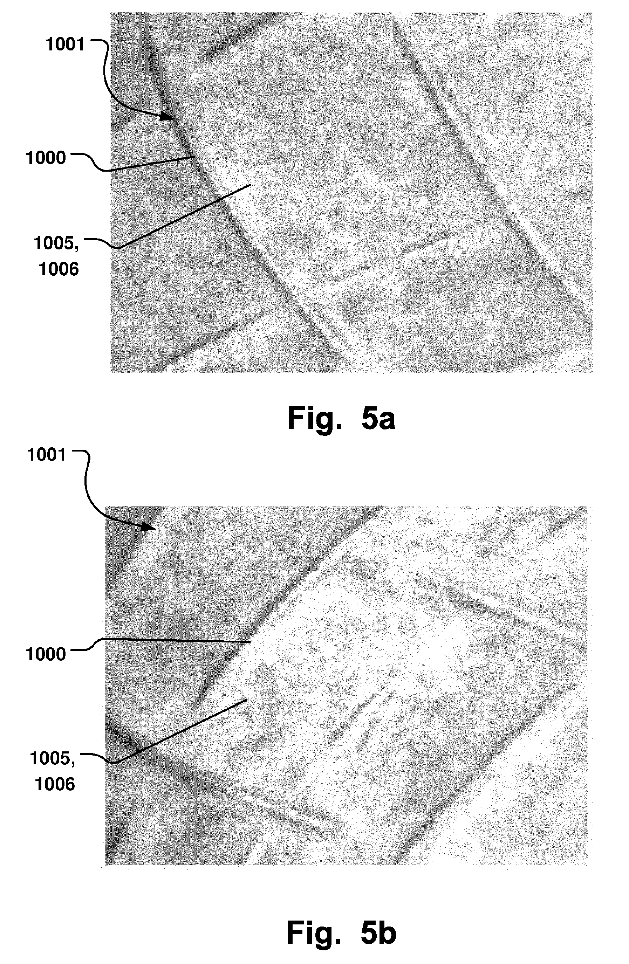

Fig. 3a-c illustrates a magnified portion of the strands of the implant 1 in Fig. 1, i.e. the cut-out section A. Fig. 3a shows three strands 1000 of the magnified portion, again with the interior 1002 and exterior surfaces 1001 indicated by line B. Fig. 3b shows the first coating 1004 applied to the strands, as in Fig. 2b, while Fig. 3c illustrates the second coating applied as a covering 1006 that bridges adjacent strands to cover the cells delimited by said strands. Figs. 4a-b and Figs. 5a-b further illustrates such covering 1006 of the second coating 1005 on implant 1. Figs. 4a-b is a viewpoint from the interior of the implant, and Figs. 5a-b viewpoint from the exterior of the implant. As seen in Figs. 4a-b the second coating 1005 is applied to the exterior surface 1001 of the strands only, as seen in the schematic Fig. 3c. The first coating 1004 is not visible in Figs. 4a-b since it comprises a microscopic layer of polymer molecules that forms an adhesion layer for the covering 1006. Non-fibrous residues of polymer molecules may be observed on the interior surface. It is thus possible to apply an occluding coating 1005 such as the covering 1006 on the external surface of the implant 1 without the use of sutures. This provides for a less time consuming manufacturing method. It is further provided for improved mechanical properties of the implant that lead to improved implant lifetime, due to the second coating being securely joined to the strands via the first coating.

Figs. 5a-b shows the second coating 1005 applied to the external surface of the strands.

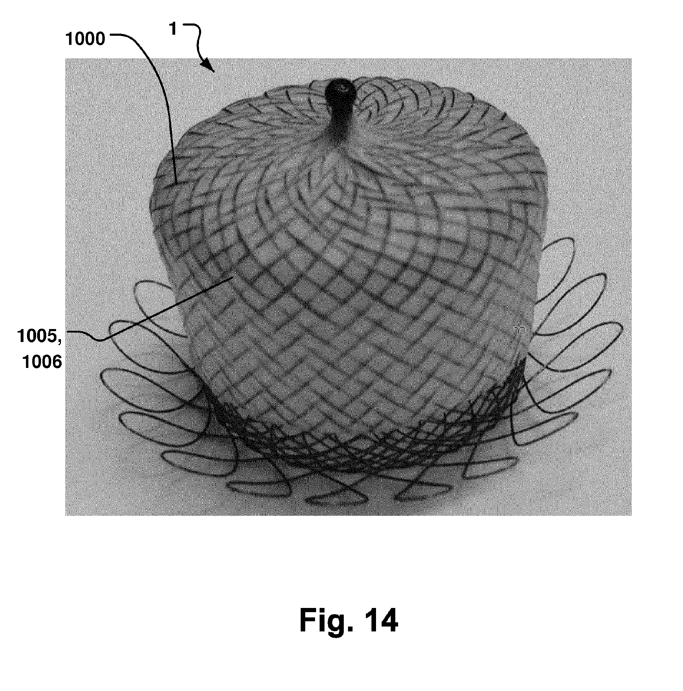

Fig. 14 is a further view of the implant 1 seen in Fig. 1. As seen in Fig. 14, the second coating 1005 forms a covering 1006 on the external surface of the implant 1, which adheres to the external surface by means of the first coating 1004 functioning as an intermediate film for improving adhesion.

The second coating may be applied 905 by a liquid solution having a higher viscosity than the liquid solution for applying said first coating. This may improve the adhesion of the second coating to the implant 1. Furthermore, by having a higher viscosity of the solution of the material from which the second coating is applied the latter can be applied to more easily cover the cells delimited by the strands as seen in Fig. 5a, while the lower viscosity of the first coating allows for formation of a more even and uniform film on the strands 1000. Hence, the second coating may be applied 912 to cover said external surface for at least partly restricting a fluid flow through said cells whereby the implant at least partly restricts a fluid flow through a structural tissue defect, such as a defect in the heart.

The first and second coating may comprises a polymer such as polyurethane, wherein the solution for applying said second coating may comprise a higher weight -% of polyurethane than the solution for applying said first coating. In some embodiments, the polymer may comprise polytetrafluoroethylene (PTFE) or expanded polytetrafluoroethylene (ePTFE).

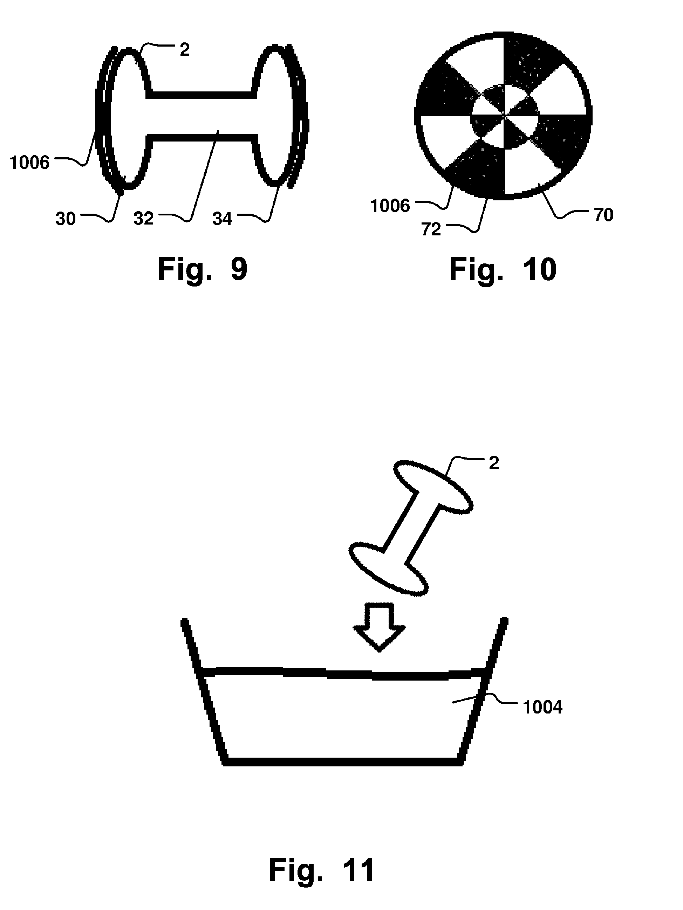

The first coating may be applied by dipping 906 the implant 1 in a liquid solution. A step of the method 900 of producing a medical implant for occluding an opening in a body is shown in Fig. 11, which is a lateral view of a medical implant 2 being coated with the first coating 1004 by dipping. The method 900 can be applied to any type of implants such as the implant 1 in Fig. 1, or any type of occluder such as an atrial septal defect (ASD) occluder, a Patent foramen ovale (PFO) occluder, a paravalvular leakage (PLD) occluder, a PDA occluder, an LAA occluder, a ventricular septal defect (VSD) occluder, or a transapical occluder. The first coating may be applied to the medical implant by dipping 906 the medical implant into a solution of a specific viscosity so that a non-fibrous coating is applied and affixed to the medical implant 1, 2. The solution may comprise a solvent that evaporates when the first coating dries.

In one embodiment only one end 13, 30, 34, and not the side 14, 32, of the medical implant 1, 2, is dipped into the solution. In another embodiment, both ends 13, 35, 30, 34, and the side 14, 32 are dipped into the solution, so as to provide the whole implant 1, 2, with coating. In yet another embodiment, only the ends 14, 35, 30, 34 of the medical implant 1, 2, are dipped into the solution, but the side 14, 32, is not dipped into the solution. Thereby, the medical implant is covered at both ends. Which parts of the medical implant 1 that are dipped into the solution may depend on what kind of medical device 1,2, is to be applied with coating, i.e. it may depend on whether the medical implant is e.g. an atrial septal defect (ASD) occluder, a Patent foramen ovale (PFO) occluder, a paravalvular leakage (PLD) occluder, a PDA occluder, an LAA occluder, a ventricular septal defect (VSD) occluder, or a transapical occluder.or some other medical implant.

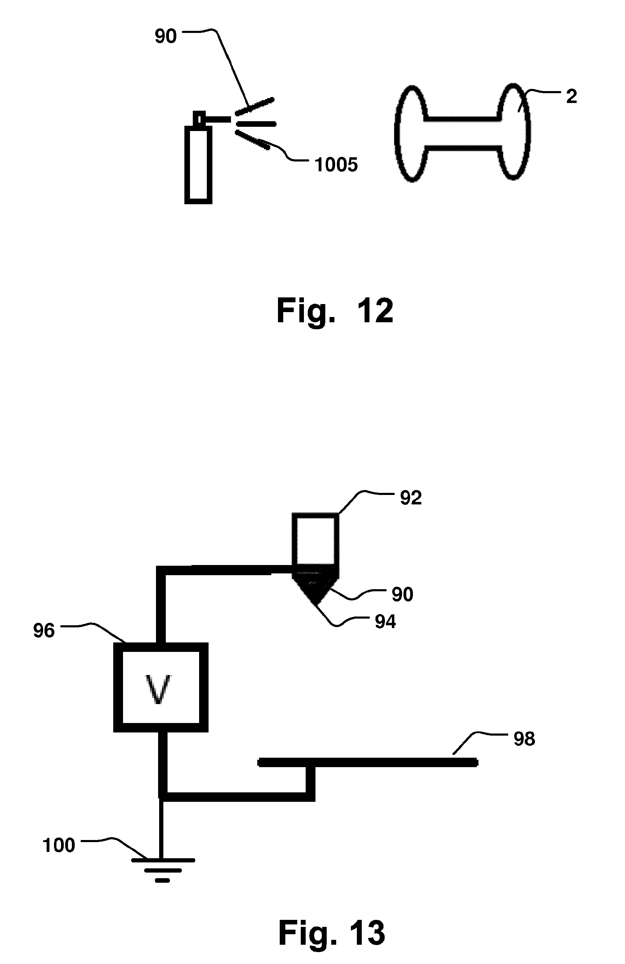

The second coating 1005 may be applied to the medical implant 1 by spraying 907 the medical implant 1, 2. The implant may be sprayed with a spray 90, which is of a specific viscosity, so that a coating 1005 is applied and affixed to an external surface of the medical implant 1, 2. This alternative is shown in Fig. 12, which is a lateral view of a medical implant 1 being coated by spraying. The solution of the material for the second coating which is sprayed may have a specific viscosity so that said second coating forms covering 1006 over the cells delimited by the strands on an external surface of said medical implant 1, 2, as seen in Figs. 3c, 4a-b, 5a-b. As mentioned above the first coating applied by dipping forms an adhesion layer for the second coating. The solution of the second coating may also comprise a solvent that evaporates and leaves the coating of the polymer on the external surface.

In one embodiment only an outer end side of a first disc-shaped section 30 of the medical implant 2 is sprayed. In another embodiment, the outer end side of the first disc-shaped section 30 and an outer end side of a second disc-shaped section 34 of the medical implant 2 are sprayed. In other embodiments, further parts of the medical implant 2 may be sprayed. As another example, substantially the whole medical implant 2 may be sprayed. Which parts of the medical implant 2 that are sprayed may depend on what kind of medical device 2 is to be applied with coating, i.e. it may depend on whether the medical implant is e.g. an atrial septal defect (ASD) occluder, a Patent foramen ovale (PFO) occluder, a paravalvular leakage (PLD) occluder, a PDA occluder, an LAA occluder, a ventricular septal defect (VSD) occluder, or a transapical occluder.or some other medical implant.

The second coating may be applied by spraying 907, electro-spinning 908, electro-spraying 909 or nano-spinning 910. Fig. 13 is a schematic sketch of a medical implant being coated by a process, such as electro-spinning or Nano-spinning. In the figure, a polymer or composite solution 90 is contained in a syringe pump 92. The syringe pump 92 comprises a spinneret, such as a hypodermic syringe needle or a metallic needle, which is connected to a high-voltage direct current power supply 96. The direct current power supply 96 is also connected to ground 100 and on the ground side, a collector 98 is connected to the direct current power supply 96. The medical implant 1, 2, to be coated would typically be placed in connection with the collector 98. The polymer solution 90 is loaded into the syringe 92 and extruded, as droplets, from the tip of the needle 94 at a constant rate by the syringe pump 92. A sufficiently high voltage must then be applied to the droplets, so that the droplets become charged. Since electrostatic repulsion counteracts the surface tension, the droplets are stretched: At a critical point, a stream of liquid erupts from the surface. This point of eruption is known as the Taylor cone. If the molecular cohesion of the liquid is sufficiently high, stream breakup does not occur and a charged liquid jet is formed. Alternatively, if stream breakup occurs, the droplets are instead electro-sprayed.

As the jet dries in flight, the mode of current flow changes from ohmic to convective as the charge migrates to the surface of the strand. The jet is then elongated by a whipping process caused by electrostatic repulsion initiated at small bends in the strand, until it is finally deposited on the grounded collector. The elongation and thinning of the strand resulting from this bending instability leads to the formation of uniform strands. Such uniform strands may have nanometer-scale diameters.

The method 900 may comprise rotating 911 the implant 1 when applying said second coating 1005. This may provide for an even and uniform covering 1006 over the external surface of the implant. This may improve the occluding ability and/or biocompatibility. Further, rotating the implant 1, 2, allows the second coating to be applied to the external surface only, as seen in Figs. 4a-b, 5a-b. As discussed above, such selectively improves on several properties of the implant, such as biocompatibility, structural integrity, flexibility etc.

The coating 11 may be made of a biocompatible and implantable material, such as PTE, PTFE or PUR.

The method 900 may further comprise heating the implant subsequent of having applied the first coating 1004. Heating the implant may lower the viscosity of the first coating. Due to the cohesion and adhesion forces, the film of the first coating on the strands is homogenized which improves the adhesion of the subsequently applied second coating. The second coating still contains solvent when being applied to the first coating, so that the previously applied film of the first coating partly resolves. This resolving of the polymer film provides for a desired reaction between the first coating, i.e. the film, and the second coating to create a strong bond.

The medical implant may be coated by a method, in which the first or second coating has been applied to the medical implant 1 in an expanded shape. The first coating and/or the second coating or any combination of the first and second coating is collectively referred to as coating 11 in some parts of the present description. By applying the coating 11 to the medical device 1, while the medical device 1 is in an expanded shape, the coating 11 is free of tension, which advantageously avoids pre-mature fatigue thereof and thus allows a reliable ingrowth. Application of a coating 11 to a medical implant 1, while the medical device 1 is in an expanded shape, may also be advantageous for other reasons, such as the fact that the medical implant 1 can be made very flexible and that a particularly large expansion/contraction ratio, i.e. a ratio of a size or diameter of the medical implant 1 in its expanded shape and the size or diameter of the medical implant 1 in its contracted shape, can be obtained for the medical implant 1.

In some embodiments, the second coating 1005, which may form a covering 1006 over the cells delimited by the strands, as seen in Figs. 3c, 4a-b, 5a-b, only covers one end 13, and not the side 14, of the medical implant 1. Some or all of these cells may be provided with the covering 1006. In another embodiment, the end 13 and the side 14 are provided with the covering 1006. The covering 1006 applied to the medical device 1 provide for an improved occlusion, improved sealing of a defect, such as a heart defect, an improved endothelialization and/or for slowing down the blood flow through the defect.

The coating 11 or covering 1006 may have an initial controllable fluid retention by perforations or microperforations thereof. The covering 1006 may cover the entire expanded diameter of the implant. Alternatively, it may only cover portions thereof. The portions may be as small as the cell structure of the fabric of the implant 1. For instance one or more cells of a braiding may be provided with a coating extending the space between adjacent strand portions forming the cells.

In this manner, different perfusion rates may be adjusted to different areas of the device. It may for instance be desired to obtain an inflow of blood into the inner of the expanded device from a distal end thereof to enhance integration of the device with surrounding blood upon clotting thereof. A reduced or prohibited outflow of blood through the proximal end may however be provided by a tighter membrane or larger diameter/surface/cells of the device being covered than those of another section of the implant 1.

The coating 1004, 1005, may alternatively be affixed to the implant 1, 2 in its collapsed shape.

Patterns of covered cells may be provided to efficiently control a desired flow pattern upon implantation. In this manner, the occlusion is not abrupt upon implantation. A certain blood flow may still occur after implantation and gradually decline upon blood coagulation and/or endotheliazation of the implanted device.

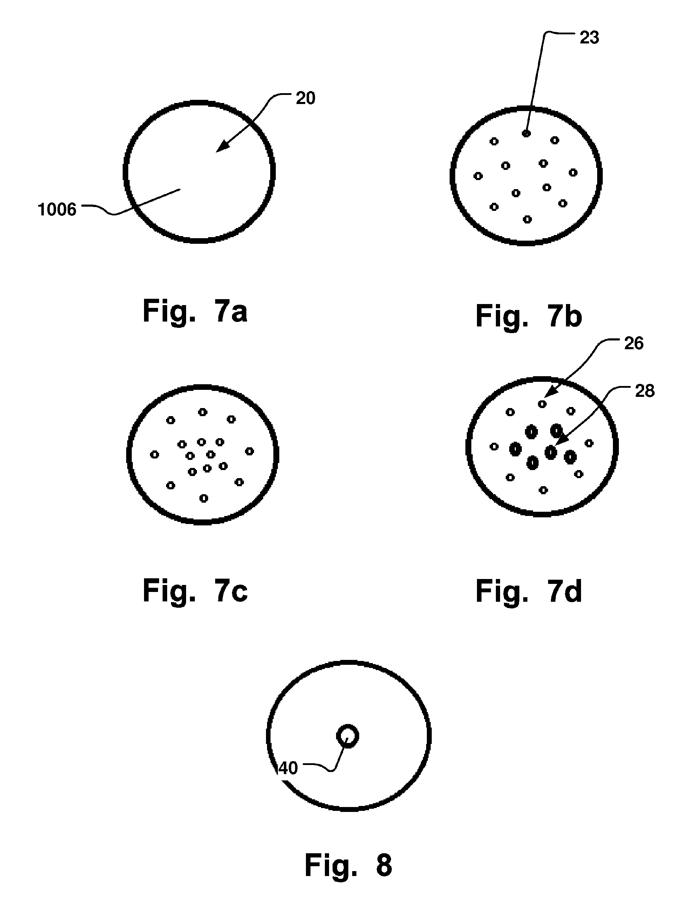

According to an embodiment, depicted in fig. 7a, one end 20 of the medical implant 1 is completely covered with a covering 1006. Thus, an improved occlusion, an improved sealing of a defect, such as a heart defect, an improved endothelialization and/or a slowing down of the blood flow through the defect is achieved. However, in order to provide some body liquid to pass through the medical implant 1 once implanted, the coating may be provided with perforations or microperforations 23. This is shown in fig. 7b, which depicts a situation where one end 20 of the medical implant 1 has been covered with a coating 22 and where the coating has been perforated, i.e. provided with perforations or microperforations. Such perforations or microperforations may be provided by a process, such as mechanical perforation or laser perforation, i.e. laser cutting. The use of laser perforation offers the advantage of a better consistency of the hole size, i.e. the perforation size, than the use of mechanical perforation. By providing the covering 1006 with perforations or microperforations, an initial controllable body liquid retention is enabled. Furthermore, the integration of the medical implant 1 may be enhanced and/or facilitated by the use of such perforations or microperforations, since the body liquid is allowed to enter into the interior of the medical implant 1. A limited blood flow may actually pass through the medical implant 1 after implantation. However, this limited blood flow will gradually decline upon blood coagulation and/or endothelialization of the implanted medical implant. Thus, by the use of perforations or microperforations, the occlusion is not abrupt, but formed gradually over time.

In one embodiment, the perforations or microperforations of the covering 1006 are uniformly distributed over the area of the covering 1006. However, in other embodiments, the perforations 24 or microperforations are randomly distributed. In yet another embodiment, depicted in fig. 7d, a first central area 28 of the covering 1006, corresponding to a first area of the medical implant is provided with perforations of a larger size, such as a diameter, than perforations of a second peripheral area 26 of the covering 1006, corresponding to a second area of the medical implant 1, so that the inflow to different areas is controlled. As an alternative, the first central area 28 of the covering 1006, corresponding to a first area of the medical implant 1, is provided with a higher number of perforations or a higher density of perforations than a second peripheral area 26 of the covering 1006, corresponding to a second area of the medical implant, so that the inflow to different areas is controlled. This is depicted in fig. 7c.

In yet another embodiment, depicted in fig. 8, the covering 1006 is arranged at an end 20 of a medical implant 1 so as to obtain an inflow of blood, into the inner of the medical implant 1, after implantation and thus in an expanded shape, from a distal end of the medical implant 1 for enhancing integration of the medical implant with surrounding blood upon clotting thereof. This can e.g. be achieved by providing the end 20 with a covering 1006, which covers substantially the whole end 20, but does not cover the section 40 of the end 20. Thus, an inflow of blood, into the inner of the medical implant 1 can be obtained through the section 40. The section 40 may be centred or situated at any other position at the end 20. As an alternative, the covering 1006 is applied only to a central portion of the end 20.

In fig. 9, another kind of medical implant 1 or occluder is shown. This medical implant 1 comprises a first disc-shaped section 30, a tubular middle section 32 and a second disc-shaped section 34. In this embodiment, the one depicted in fig. 9, only the ends of the medical implant 1 are coated or provided with the covering 1006, i.e. the outer end side of the first disc-shaped section 30 and the outer end side of the second disc-shaped section 34 are coated or provided with the covering 1006. In some embodiments, the tubular middle section 32 is provided with the covering 1006, whereas the disc-shaped sections 30, 34 are not provided with a coating.

Fig. 10 is a top view of a medical implant, provided with a covering 1006, wherein the covering 1006 forms a pattern. Such a pattern can be any pattern, which is advantageous for control of a desired flow pattern through the medical implant 1 upon implantation. In the embodiment according to fig. 10, the medical implant 1 is provided with a covering 1006 in some sections, i.e. one section 72 of the end portion 20 of the medical implant 1 is provided with a covering 1006, whereas all adjacent sections 70 are not provided with a coating and likewise all sections being adjacent to a non-coated section 70 are provided with a covering 1006. By the use of such a pattern of covered sections 72 or cells an efficient control of a desired flow pattern through the medical implant 1 upon implantation is established. The sections or cells may be large and thus cover large portions of the medical implant or as small as a gap between adjacent strands of the mesh, which makes up the medical implant 1. The pattern may be formed be first applying a covering 1006, i.e. second coating 1005 by a method 900 as described above, and thereafter removing parts of the covering 1006 so as to form the pattern.

The present disclosure has been described above with reference to specific embodiments. However, other embodiments than the above described are equally possible within the scope of the disclosure. Different method steps than those described above, may be provided within the scope of the disclosure. The different features and steps of the disclosure may be combined in other combinations than those described. The scope of the disclosure is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used.

## Claims

1. A method (900) of producing a medical implant (1) for occluding an opening in a body, said method comprising:
braiding, knitting or weaving (901) together strands (1000) to form a body mesh of strands and forming a plurality of adjacent cells delimited by said strands,
and
applying (902) a first coating (1004) to said strands, applying (904) a second coating (1005) to at least part of an external surface (1001) of said medical implant (1), whereby the ratio between the amount of the first and second coating on said external surface is different from said ratio on an internal surface (1002) of the implant.

2. Method according to claim 1, wherein said second coating is applied to said external surface only.

3. Method according to claim 1 or 2, wherein said second coating is applied subsequent of applying said first coating.

4. Method according to any of claims 1-3, wherein said first coating comprises a polymer and whereby drying said first coating leaves a residual non-fibrous layer (1003) of polymer molecules on said implant for subsequent adhesion to said second coating.

5. Method according to any of claims 1-4, wherein said second coating is applied (905) by a liquid solution having a higher viscosity than the liquid solution for applying said first coating.

6. Method according to any of claims 1-5, wherein said first and second coating comprises a polymer such as polyurethane, wherein the solution for applying said second coating comprises a higher weight -% of polyurethane than the solution for applying said first coating.

7. Method according to any of claims 1-6, wherein said first coating is applied by dipping (906) said implant in a liquid solution.

8. Method according to any of claims 1-7, wherein said second coating is applied by spraying (907), electro-spinning (908), electro-spraying (909) or nano-spinning (910).

9. Method according to any of claims 1-8, comprising rotating (911) said implant when applying said second coating.

10. Method according to any of claims 1-9, wherein said second coating forms a covering (1006) over said cells on an external surface of said medical implant.

11. Method according to any of claims 1-10, wherein said second coating is applied to said medical implant (1) by spraying (907) said medical implant (1) with a spray having a specific viscosity so that said second coating forms covering (1006) over said cells on an external surface of said medical implant (1).

12. A medical implant (1), such as an atrial septal defect (ASD) occluder, a Patent foramen ovale (PFO) occluder, a paravalvular leakage (PLD) occluder, a PDA occluder, an LAA occluder, a ventricular septal defect (VSD) occluder, or a transapical occluder, for occluding an opening in a body, wherein said medical implant (1) comprises
a body mesh of strands (1000), said strands being braided, knitted or woven together to form the mesh, said body mesh forming a plurality of adjacent cells (1007) delimited by said strands, said body mesh having an external surface (1001), and
a first coating (1004) on said strands, and
a second coating (1005) covering at least part of said external surface for at least partly restricting a fluid flow through a structural tissue defect, such as a defect in the heart, whereby the ratio between the amount of the first and second coating on said external surface is different from said ratio on an internal surface of the implant.

13. Medical implant according to claim 12, wherein said second coating only covers said external surface of the implant.

14. Medical implant according to claim 12, wherein said first coating provides a binding force to said second coating such that said second coating adheres to said strands.

15. Medical implant according to any of claims 12 to 14, wherein said firs or second coating comprises a polymer, such as Polyurethane (PU), Polytetrafluoroethylene (PTFE) or Expanded Polytetrafluoroethylene (ePTFE).
